(19) Europäisches Patentamt
European Patent Office
Office européen des brevets

(11) Veröffentlichungsnummer: **0 053 221**
**B1**

(12) # EUROPÄISCHE PATENTSCHRIFT

(45) Veröffentlichungstag der Patentschrift: **21.08.85**

(51) Int. Cl.⁴: **A 61 L 15/00**, D 04 H 1/00

(21) Anmeldenummer: **81104638.2**

(22) Anmeldetag: **16.06.81**

(54) **Saugfähige Wegwerfartikel.**

(30) Priorität: **27.11.80 DE 3044631**

(43) Veröffentlichungstag der Anmeldung: **09.06.82 Patentblatt 82/23**

(45) Bekanntmachung des Hinweises auf die Patenterteilung: **21.08.85 Patentblatt 85/34**

(84) Benannte Vertragsstaaten: **BE DE FR GB NL**

(56) Entgegenhaltungen:
**EP-A-0 009 977**
**DE-A-1 902 477**
**DE-A-2 722 860**
**DE-A-2 740 184**

**Die Akte enthält technische Angaben, die nach dem Eingang der Anmeldung eingereicht wurden und die nicht in dieser Patentschrift enthalten sind.**

(73) Patentinhaber: **Firma Carl Freudenberg, Höhnerweg 2, D-6940 Weinheim/Bergstrasse (DE)**

(72) Erfinder: **Hartmann, Ludwig, Dr., Pascalstrasse 3, D-6750 Kaiserslautern (DE)**
Erfinder: **Ruzek, Ivo, Am Harzhübel 56, D-6750 Kaiserslautern (DE)**

(74) Vertreter: **Weissenfeld-Richters, Helga, Dr., Höhnerweg 2, D-6940 Weinheim/Bergstrasse (DE)**

**EP 0 053 221 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft einen saugfähigen Wegwerfartikel, insbesondere zur Verwendung auf dem Gebiet der Medizin und Hygiene, bestehend aus einer Zellulose enthaltenden Saugschicht und einer darauf angeordneten Abdeckschicht aus synthetischen Fasern, die zur Verbesserung des Primärtransportes der aufzusaugenden Flüssigkeit in Richtung der Saugschicht mit einem Netzmittel versehen ist.

Saugfähige Wegwerfartikel der vorgenannten Art sind bekannt. Sie gelangen auf diversen hygienischen und medizinischen Gebieten zur Anwendung. So werden beispielsweise Einwegwindeln aus einer saugfähigen Schicht, die im wesentlichen aus Zellstoff besteht, oberflächlich mit einem gut deckenden und festen Vliesstoff aus synthetischen Fasern abgedeckt. Ähnlich aufgebaut sind auch Binden und Tampons für die Damenhygiene und chirurgische Tampons. Weiterhin sind mehrschichtige Flächengebilde (Composites) bekannt, bei denen saugfähige Lagen nach außen hin mit einem Vliesstoff abgedeckt werden, wobei die Mehrschichtgebilde als Einweghandtücher, Wischtücher usw. im Haushalt, in der Industrie oder im klinischen Bereich verwendet werden. Schließlich werden solche Vliesstoffe auch als Materialien zur Herstellung von Einwegkleidung für den klinischen Gebrauch oder aber auch für andere Zwecke verwendet.

Vliesstoffe, die als Abdeckmaterialien bei solchen Wegwerfartikeln zur Anwendung gelangen, müssen mechanisch hinreichend fest sein, eine genügende Opazität und Konfektionsfähigkeit aufweisen. Daneben ist es wesentlich, daß die Abdeckvliesstoffe einen hinreichenden Transport wäßriger Flüssigkeiten oder Dämpfe in die saugfähigen Schichten zulassen. Beide Bedingungen sind bei der Abdeckschicht des eingangs beschriebenen Wegwerfartikels nach der DE-OS 2 740 184 erfüllt. Die Dochtwirkung der Abdeckschicht ist jedoch durch das enthaltene Netzmittel sowohl in Richtung der Saugschicht als auch in Richtung der Oberfläche wirksam. Letztere fühlt sich daher nach einer Durchnässung der Saugschicht ebenfalls naß an. Das ist nicht erwünscht.

Unter Dochtwirkung wird die Fähigkeit der Abdeckschicht verstanden, durch Flüssigkeit benetzbar zu sein und gleichzeitig die Flüssigkeit infolge Kapillarwirkung weiterzuleiten. Ist die Dochtwirkung nicht hinreichend, so ist der Abtransport der Flüssigkeit nicht gewährleistet und die Saugeigenschaften des Erzeugnisses sind mangelhaft.

Bei hygienischen und medizinischen Mehrschichtgebilden genügt in der Regel eine Dochtwirkung, ähnlich der bei dem Wegwerfartikel nach der DE-OS 2 740 184 nicht, denn es ist notwendig, daß der Rücktransport der anschließend in der Saugschicht gespeicherten Flüssigkeit nach außen weitgehend verhindert wird, so daß ein »trockener« Eindruck entsteht. Die Dochtwirkung in umgekehrter Richtung muß somit reduziert, wenn nicht gänzlich verhindert werden. Das bedeutet, daß die beiden Forderungen — der Primärtransport und anschließend die Sperrwirkung — an sich in einem krassen Widerspruch zueinander stehen.

Der Erfindung liegt nun die Aufgabe zugrunde, einen saugfähigen Wegwerfartikel zu entwickeln, der beide Forderungen optimal erfüllt. Die Flüssigkeit soll sehr rasch in die saugfähige Schicht abgeleitet werden, wobei ein Rücktransport nicht oder nur unwesentlich erfolgt.

Diese Aufgabe wird erfindungsgemäß bei einem Wegwerfartikel der eingangs genannten Art dadurch gelöst, daß das Netzmittel von der aufzusaugenden Flüssigkeit aus der Abdeckschicht heraus und in die Saugschicht transportierbar ist und daß das Netzmittel von Zellulose chemosorbierbar ist, um nach Abschluß des Primärtransportes eine Sperrwirkung in umgekehrter Richtung zu erzielen. Überraschend wurde festgestellt, daß man die völlig widersprüchlichen Forderungen eines raschen Primärtransportes zum Saugpolster hin und eine Sperrwirkung in umgekehrter Richtung in dieser Weise optimal erfüllt.

Für eine gute Dochtwirkung ist es erforderlich, daß die Vliesoberfläche bzw. die Oberfläche der den Vliesstoff bildenden Fasern zunächst durch die Flüssigkeit benetzt wird. Bekanntlich verfügen nun die meisten synthetischen Polymeren über eine relativ geringe Oberflächenenergie, wogegen Wasser und die meisten wäßrigen Flüssigkeiten durch eine relativ hohe Grenzflächenenergie gekennzeichnet sind ($72{,}7 \times 10^{-3}$ N/m). Die Grenzflächenenergie der wichtigsten faserbildenden Polymeren gibt die folgende Tabelle wieder:

| | Grenzflächenenergie ($10^{-3}$ N/m) kritische | davon polarer Anteil | disperser Anteil |
|---|---|---|---|
| Polyethylenterephthalat | 47,3 | 4,1 | 43,2 |
| Polyamid | 47,0 | 6,2 | 40,8 |
| Polypropylen | 33,2 | 0,0 | 33,2 |

Als kritische Grenzflächenenergie wird dabei der Wert der Grenzflächenenergie angegeben, den eine Flüssigkeit höchstens erreichen kann und dabei die Oberfläche noch benetzt. Als disperser Anteil der Grenzflächenenergie wird der Teil der gesamten Grenzflächenenergie bezeichnet, der nur von Dispersionskräften herrührt, wogegen als polarer Anteil derjenige Anteil genannt ist, der ausschließlich auf den polaren Wechselwirkungen beruht. Beim Wasser wird angenommen, daß der überwiegende Teil — etwa $^2/_3$ — auf die polaren und nur etwa $^1/_3$ auf die dispersen Wechselwirkungen zurückzuführen ist.

Die Dochtwirkung hängt von der Kapillarität des porösen Materials und von seiner Oberflächenenergie in Relation zu derjenigen der zu transportierenden Flüssigkeit ab. Ein Unterschied der Oberflächenenergien wirkt sich wie eine Verkleinerung der Porosität aus. Bei einem vorgegebenen porösen Material kann man somit die Dochtwirkung durch Erhöhung des kritischen Grenzflächenenergiewertes und seine Anpassung an den Wert der zu transportierenden wäßrigen Flüssigkeit verbessern. Dies läßt sich z. B. durch Behandlung der Vlies- bzw. Faseroberfläche mit Tensiden erreichen. Als wirksame Tenside, die eine hinreichende Adsorptionsfähigkeit auf den Polymeroberflächen besitzen, kommen an sich bekannte nichtionogene Tenside in Frage.

Sehr wirksam sind ethoxylierte Alkylarylverbindungen, deren Wirkung durch entsprechende Variation der Zusammensetzung, dem jeweiligen Verwendungszweck, angepaßt einstellbar ist. Durch die Verwendung derartiger Tenside läßt sich eine Verbesserung der Benetzbarkeit der Vliesoberfläche erzielen und der Flüssigkeitstransport durch den Vliesstoff hindurch wird beschleunigt. Dies rührt daher, daß durch die Angleichung der Oberflächenenergien des Vliesstoffes und der zu transportierenden Flüssigkeit wie eine effektive Erhöhung der Porosität wirkt. Wird einmal die vollständige Benetzung der Oberfläche erreicht, so kann die Flüssigkeit durch die Kapillaren der Saugschicht zugeleitet werden. In der Saugschicht wird die Flüssigkeit dann gespeichert.

Obgleich die Dochtwirkung eines so behandelten Vliesstoffes sehr gut ist, ergeben sich schwerwiegende Mängel wegen des ungehinderten Rücktransportes der Flüssigkeit, wenn man auf die Oberfläche des mit einem derartigen Vliesstoff abgedeckten Saugpolsters drückt. Der Abdeckvließsstoff wird sofort wieder mit der in dem Saugpolster gespeicherten Flüssigkeit benetzt. Er erscheint naß und es kommt zum nahezu ungehinderten Flüssigkeitsrücktransport in entgegengesetzter Richtung. Die verbesserte Benetzbarkeit, die dem ursprünglichen Flüssigkeitstransport in der erwünschten Richtung zum Saugpolster hin behilflich war, ist nun nachteilig und unerwünscht. Nichtionogene Tenside, wie sie üblicherweise als Netzmittel industriell verwendet werden, ermöglichen somit zwar eine Dochtwirkung, jedoch erweisen sie sich für die Ausrüstung von Abdeckvliesen als ungeeignet, denn sie bleiben an der Vlies- bzw. Faseroberfläche adsorbiert und beschleunigen dadurch den Flüssigkeitstransport in beiden Richtungen. Die erfindungsgemäß gestellte Aufgabe wird somit mit üblichen Tensiden nicht gelöst.

Nichtionogene Tenside gehen, wenn überhaupt, dann nur zu einem sehr geringen Teil in die flüssige Phase über. Auch dort sorgen sie für die Aufrechterhaltung der Benetzungswirkung in beiden Richtungen in vollem Umfange. Will man den Rücktransport der gespeicherten Flüssigkeit reduzieren, so muß man durch eine feine Einstellung der Tensidmenge einen Gleichgewichtszustand anstreben, bei dem sowohl der primäre Transport zum Saugpolster hin als auch der Rücktransport berücksichtigt werden muß. Es ist allenfalls durch eine Kompromißlösung möglich, ein annähernd zufriedenstellendes Ergebnis zu erzielen.

Verwendet man jedoch Tenside, welche an der Zellulose bzw. dem Zellulose enthaltenden Material des Saugpolsters chemosorbierbar sind und die durch die Flüssigkeit in das Saugpolster transportierbar sind, so wird die Aufgabe in optimaler Weise gelöst. Es wurde festgestellt, daß die an sich völlig widersprüchlichen Forderungen eines raschen Primärtransportes zum Saugpolster hin und einer Sperrwirkung umgekehrter Richtung sich so gut vereinbaren lassen. In ganz besonderem Maße haben sich als Netzmittel für den Abdeckvliesstoff kationenaktive Tenside der allgemeinen Formel

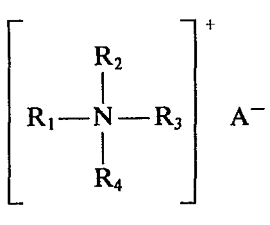

bewährt, wobei $R_1$, $R_2$, $R_3$ und $R_4$ Alkyl-, Aryl-, Alkylaryl-, Ethoxyl-, Alkylethoxyl-, Arylethoxyl- und/oder Alkylarylethoxyl-Radikale sein können. Der Rest A bedeutet ein übliches Anion, z. B. ein Halogenid.

Es wurde gefunden, daß die kationenaktiven Tenside eine hervorragende Netzwirkung aufweisen. Sie verbessern die Dochtwirkung wesentlich, weil sie einerseits nur schwach von der Polymeroberfläche adsorbiert werden, so daß ein wesentlicher Teil während der Benetzung in die flüssige Phase übergeht und daß sie sich andererseits substantiv gegenüber Zellulose verhalten, d. h. sie werden auf Zellulose enthaltenden Substraten chemosorbiert und dadurch der flüssigen Phase wieder entzogen.

Die flüssige Phase dient somit als Transportmittel des Netzmittels von der an sich wasserabweisenden Faser in das saugfähige Medium hinein. Der Abdeckvliesstoff kann deshalb zur Verbesserung des Primärtransportes gut benetzt werden, weil eine hinreichende Menge des Tensids verwendet werden kann. Durch die Abwanderung des kationenaktiven Tensids und die anschließende Bindung an das Zellulose enthaltende Substrat wirkt wie eine Sperrschicht für den unerwünschten Rücktransport der Flüssigkeit. Da der aus synthetischen Fasern bestehende Vliesstoff selbst nur sehr wenig Wasser aufnimmt bzw. bindet, erscheint er »trocken«, selbst wenn er über ein feuchtes Saugpolster verspannt ist.

Die erfindungsgemäß vorgeschlagenen kationenaktiven Tenside können hoch dosiert werden. So ist es möglich, eine rasche Benetzung zu erzielen, ohne daß dabei der Rücktransport gefördert wird. Man erhält optimale Eigenschaften des Abdeckvließstoffes im Hinblick auf die sehr widersprüchlichen Forderungen der Docht- und Sperrwirkung.

Die vorgeschlagenen kationenaktiven Tenside haben überdies den Vorteil, daß sie bakteriostatisch bzw. sogar bakterizid sind, was für viele Anwendungsgebiete insbesondere im medizinischen Bereich sehr erwünscht ist. Erfindungsgemäß ausgerüstete Abdeckvliesstoffe sind im klinischen und medizinischen Bereich sowie auch im kosmetisch-hygienischen Bereich unbedenklich einsetzbar. Hierbei ist es wichtig, daß die Tenside keine negativen Nebenwirkungen auf den menschlichen Organismus haben.

Unter den vorgeschlagenen kationenaktiven Tensiden haben sich folgende Verbindungen als besonders zweckmäßig erwiesen:

Stearyldimethylbenzylammoniumchlorid:

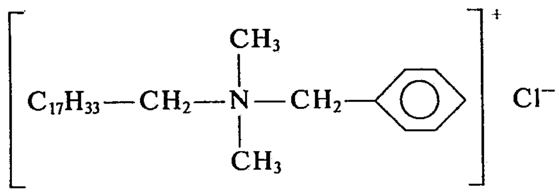

Methyldodecylbenzyltrimethylammoniumchlorid:

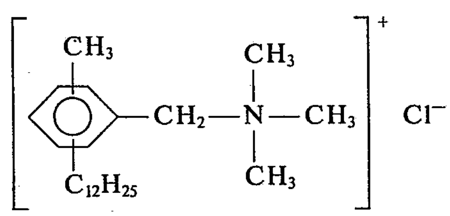

Dodecyldimethylbenzylammoniumchlorid:

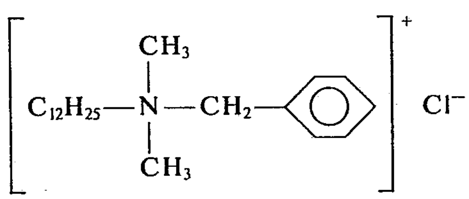

Tetradecyldimethylbenzylammoniumchlorid:

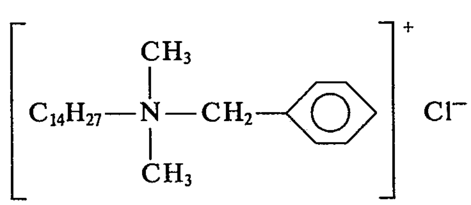

Hexadecyldimethylbenzylammoniumchlorid:

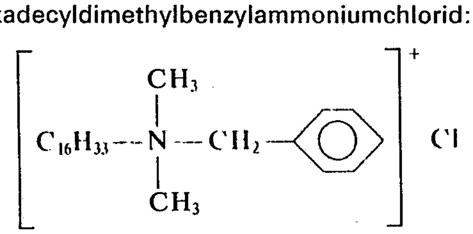

Diisobutylcresoxyethoxyethyldimethylbenzammoniumchlorid:

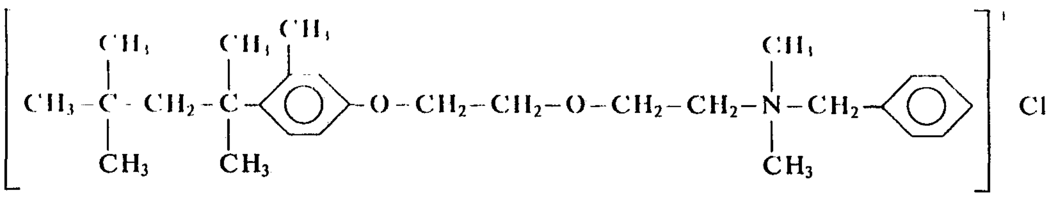

Diisobutylphenoxyethoxyethyldimethylbenzylammoniumchlorid:

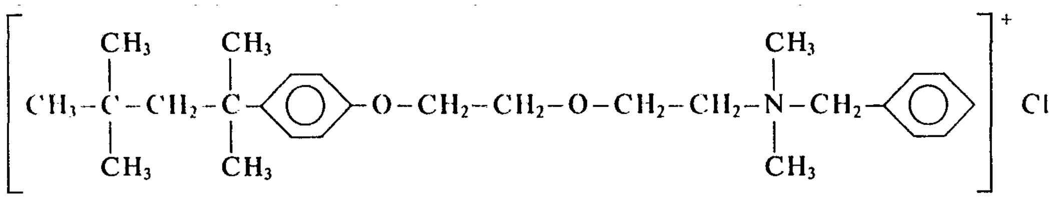

Methyldodecylxylylen-bis-(trimethylammoniumchlorid):

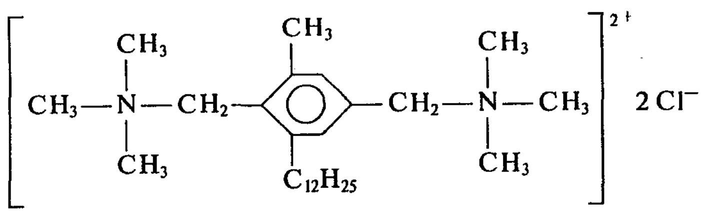

Obgleich beliebig hohe Mengenanteile kationenaktiver Tenside eingesetzt werden können, haben sich im allgemeinen zur Ausrüstung von Abdeckvliesen Auftragsmengen von 0,05 bis 1,0 Gew.-%, bezogen auf das Vliesstoffgewicht, bewährt. Vielfach genügen Mengen bis zu 0,5 Gew.-%.

Die Ausrüstung des Vliesstoffes kann in an sich bekannter Weise durch Foulardieren, Pflatschen oder Aufsprühen der wäßrigen Lösungen oder Dispersionen und anschließende Trocknung, gegebenenfalls unter Druck, erfolgen. Vliesstoffe jeglicher Zusammensetzung lassen sich so behandeln. Die kationenaktiven Tenside lassen sich auch üblichen Avivagen zumischen und so in besonders einfacher Weise einsetzen. Eine besonders vorteilhafte Ausrüstungsmöglichkeit besteht darin, die kationenaktiven Tenside in entsprechender Dosierung der Polymerschmelze während des Spinnvorganges zuzumischen. Die kationenaktiven Tenside stellen dabei eine der Polymerschmelze fremde und mit ihr nicht mischbare Phase dar, die aufgrund ihrer niederen Viskosität zu der Faseroberfläche hin transportiert wird.

## Beispiel 1

Ein Spinnvliesstoff aus Polypropylenfäden eines Filamenttiters von 2,2 dtex von einem Flächengewicht von 15 g/m$^2$, gebunden nach der Punktkalandertechnik, der eine Dicke von 0,16 mm aufweist, wird durch Tränkung mit einer wäßrigen Lösung von Diisobutylphenoxyethoxyethyldimethylbenzylammoniumchlorid als kationenaktives Tensid ausgerüstet. Die Auftragsmenge wird durch die Konzentration der Lösung und durch die aufgetragene Flüssigkeitsmenge eingestellt, wie dies aus der Tabelle hervorgeht. Anschließend wird bei 100° C in einem Lufttrockner getrocknet.

Der ausgerüstete Vliesstoff wird nach dem folgend beschriebenen Verfahren auf die Dochtwirkung (run-off) und auf die Wiederbenetzung (rewet) geprüft. Die Ergebnisse sind in der Tabelle wiedergegeben.

## Dochtwirkung (run-off) Test

Ein 300 g/m$^2$ schweres Zellstoffsaugpolster von einer Länge von mindestens 250 mm wird auf einer glatten undurchlässigen Unterlage mit dem zu prüfenden Abdeckvliesstoff überspannt und unter einem Winkel von 45° zur Senkrechten angeordnet. Am oberen Ende des so hergestellten Saugpolsters wird in einer Entfernung von 200 mm vom unteren Ende eine Auslaufdüse angebracht, die mit einer automatischen Pipette mit einem Volumen von 30 ml verbunden wird. Die Auslaufdüse wird so konstruiert, daß sie in der beschriebenen Anordnung einen Durchfluß von 0,5 ml/sec sichert. Unter der

Unterkante des Saugpolsters werden mehrere Lagen von Filterpapier zur Abfangung der Überschußmenge angeordnet. Danach werden aus der Pipette die 30 ml der Prüfflüssigkeit während 60 sec auf das Saugpolster aufgegossen. Als Prüfflüssigkeit wird destilliertes Wasser, dessen Grenzflächenspannung durch Zugabe von dem kationenaktiven Tensid auf $42 \times 10^{-3}$ N/m bei 23°C eingestellt wird, verwendet. Der Flüssigkeitsstrom fließt zunächst an der Oberfläche des Abdeckvliesstoffes hinab, bis es zu einer Benetzung kommt. Daraufhin wird der Rest der Prüfflüssigkeit durch das Saugpolster aufgesaugt und gespeichert. Der Überschuß der Prüfflüssigkeit, der die Unterkante des Saugpolsters erreicht, wird von dem untergestellten Filterpapier aufgesaugt und durch Differenzwegen erfaßt. Ein gutes Abdeckvlies soll zu einem Überschuß von höchstens 0,5 g führen. Die Dichtwirkung ist um so besser, je geringer der an der Unterkante aufgefangene Überschuß ist.

### Wiederbenetzungs-(rewet) Test

Ein Zellstoffsaugpolster von einem Flächengewicht von 300 g/m² wird über eine glatte, waagerecht liegende Platte gespannt und mit dem zu prüfenden Vliesstoff abgedeckt. Auf das Saugpolster wird ein 20 mm langes Rohr von einem Durchmesser von 20 mm, an dessen Unterseite sich ein Sieb zur Verteilung der Flüssigkeit befindet, aufgesetzt. Dann werden 30 ml der in dem vorstehend beschriebenen run-off Test angegebenen Prüfflüssigkeit eingefüllt. Nachdem die Flüssigkeit durch das Saugpolster aufgesaugt ist, wird die benetzte Stelle mit einem walzenförmigen Metallkörper von einem Gewicht von 30 N und einer Kontaktfläche von 100 cm² 3 Minuten lang belastet. Anschließend wird unter den Metallkörper ein mehrfach zusammengefaltetes Filterpapier gelegt und die benetzte Stelle weitere 2 Minuten lang belastet. Die durch den Abdeckvliesstoff durchdringende Flüssigkeit wird von dem Filterpapier aufgesaugt und über Differenzwegen erfaßt. Das Ergebnis wird als Wiederbenetzung (rewet) bezeichnet und soll bei einem noch als »trocken« zu bezeichnenden Vliesstoff geringer als 1,0 g sein.

### Beispiel 2

Unter den Bedingungen des Beispiels 1 wird als kationenaktives Tensid ein Gemisch von 80 Gew.-% Methyldodecylbenzyltriammoniumchlorid und 20 Gew.-% Methyldodecylxylylen — bis — (trimethylammoniumchlorid) verwendet. Die Ergebnisse sind wiederum in der Tabelle angeführt.

### Vergleichsbeispiel

Unter den Bedingungen der Beispiele 1 und 2 wird anstelle des erfindungsgemäßen kationenaktiven Tensids ein handelsübliches nichtionogenes Tensid verwendet. Das Ergebnis ist ebenfalls in der Tabelle wiedergegeben.

Tabelle 1

| | Tensidauftrag in mg/Tensid/m² | Dochtwirkung (run-off) in g | Wiederbenetzung (rewet) in g |
|---|---|---|---|
| Beispiel 1 | | | |
| a | 25 | 0,17 | 0,18 |
| b | 50 | 0,12 | 0,22 |
| Beispiel 2 | | | |
| a | 25 | 0,21 | 0,17 |
| b | 50 | 0,02 | 0,26 |

Tabelle 1 (Fortsetzung)

| | Tensidauftrag in mg/Tensid/m² | Dochtwirkung (run-off) in g | Wiederbenetzung (rewet) in g |
|---|---|---|---|
| Vergleichsbeispiel | | | |
| a | 25 | 0,65 | 0,70 |
| | 50 | 0,20 | 1,80 |
| | 75 | 0,14 | 2,20 |

**Patentansprüche**

1. Saugfähiger Wegwerfartikel; insbesondere zur Verwendung auf dem Gebiet der Medizin und Hygiene, bestehend aus einer Zellulose enthaltenden Saugschicht und einer darauf angeordneten Abdeckschicht aus synthetischen Fasern, die zur Verbesserung des Primärtransportes der aufzusaugenden Flüssigkeit in Richtung der Saugschicht mit einem Netzmittel versehen ist, dadurch gekennzeichnet, daß das Netzmittel von der aufzusaugenden Flüssigkeit aus der Abdeckschicht heraus und in die Saugschicht transportiert ist, und daß das Netzmittel von Zellulose chemosorbierbar ist, um nach Abschluß des Primärtransportes eine Sperrwirkung in umgekehrter Richtung zu erzielen.

2. Wegwerfartikel nach Anspruch 1, dadurch gekennzeichnet, daß er als Netzmittel ein kationenaktives Tensid der allgemeinen Formel

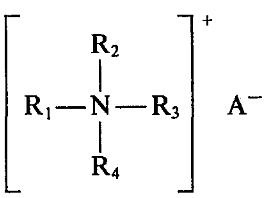

wobei $R_1$ bis $R_4$ Alkyl-, Aryl-, Alkylaryl-, Ethoxyl-, Alkylethoxyl-, Arylethoxyl- und/oder Alkylarylethoxyl-Radikale sein können und A ein geeignetes Anion, z. B. ein Halogenid.

3. Wegwerfartikel nach einem der Ansprüche 1 oder 2, dadurch gekennzeichnet, daß das Netzmittel in einer Menge von 0,05 bis 1,0 Gew.-%, bezogen auf das Vliesstoffgewicht, verwendet ist.

4. Verfahren zur Herstellung eines Wegwerfartikels nach einem der Ansprüche 1 bis 3, durch Abdecken einer Zellulose enthaltenden Saugschicht mit einem oberflächenbehandelten Vliesstoff, dadurch gekennzeichnet, daß der Vliesstoff nach einem an sich bekannten Spinnverfahren hergestellt wird, bei dem das kationenaktive Tensid der Polymerschmelze beigemischt wird und zusammen mit dieser ausgesponnen wird, wobei das Tensid während des Spinnvorgangs und der anschließenden Verfestigung der Fasern bzw. Fäden insbesondere an deren Oberfläche angereichert wird.

**Claims**

1. An absorbent disposable article, particularly for use in the field of medicine and hygiene, comprising a cellulose-containing absorbent layer and, arranged thereon, a covering layer which consists of synthetic fibres and has been provided with a wetting agent to improve the primary of the liquid to be absorbed in the direction of the absorbent layer, characterised in that the wetting agent is transportable out of the covering layer and into the absorbent layer by the liquid to be absorbed, and in that the wetting agent is chemosorbable by cellulose to produce a blocking action in the reverse direction after conclusion of the primary transport.

2. A disposable article according to claim 1, characterised in that the wetting agent is a cationic surfactant of the general formula

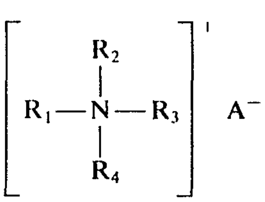

where $R_1$ to $R_4$ are selected from alkyl, aryl, alkylaryl, ethoxyl, alkylethoxyl, arylethoxyl and alkylarylethoxyl radicals and A is an appropriate anion, for example a halide.

3. A disposable article according to claim 1 or 2, characterised in that the wetting agent is used in an amount of 0.05 to 1.0% by weight, based on the weight of nonwoven.

4. A process for preparing a disposable article according to any one of claims 1 to 3 by covering a cellulose-containing absorbent layer with a surface-treated nonwoven, characterised in that the nonwoven is prepared by a spinning method known per se in which the cationic surfactant is admixed with the polymer melt and is spun out together with the latter, the surfactant being concentrated in the course of the spinning process and the subsequent consolidation of the fibres or filaments, in particular at their surface.

## Revendications

1. Article absorbant à jeter destiné à être utilisé, en particulier, dans le domaine de la médecine et de l'hygiène, cet article étant constitué d'une couche absorbante contenant de la cellulose, ainsi que d'une couche de recouvrement disposée sur cette couche absorbante, constituée de fibres synthétiques et comportant un agent mouillant en vue d'améliorer le transport primaire du liquide à absorber en direction de la couche absorbante, caractérisé en ce que l'agent mouillant peut être transporté par le liquide à absorber hors de la couche de recouvrement pour être amené dans la couche absorbante, tandis qu'il peut subir une chimisorption par la cellulose afin d'obtenir, au terme du transport primaire, un effet d'arrêt en sens inverse.

2. Article à jeter selon la revendication 1, caractérisé en ce que, comme agent mouillant, il contient un agent tensio-actif cationique actif de formule générale:

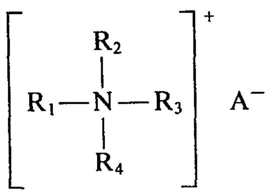

dans laquelle les radicaux $R_1$ à $R_4$ peuvent être des groupes alkyle, aryle, alkylaryle, éthoxy, alkyl-éthoxy, aryléthoxy et/ou alkylaryléthoxy, tandis que A est un anion approprié, par exemple, un halogénure.

3. Article à jeter selon une des revendications 1 et 2, caractérisé en ce qu'on utilise l'agent mouillant en une quantité de 0,05 à 1% en poids, calculé sur le poids du voile.

4. Procédé de fabrication d'un article à jeter selon une des revendications 1 à 3 en recouvrant une couche absorbante contenant de la cellulose avec un voile traité en surface, caractérisé en ce que le voile est réalisé selon un procédé de filage connu en soi dans lequel l'agent tensio-actif cationique actif est ajouté à la masse fondue du polymère et est filé conjointement avec cette dernière, l'agent tensio-actif s'accumulant au cours du processus de filage et de la consolidation ultérieure des fibres ou des fils, en particulier, sur leur surface.